# EUROPEAN PATENT SPECIFICATION

(11) **EP 1 272 854 B1**
(45) Date of publication and mention of the grant of the patent: **03.03.2004**
(21) Application number: 01922136.5
(22) Date of filing: 09.04.2001
(51) Int. Cl.: G01N 33/74, G01N 33/78, G01N 33/58, G01N 33/543

(54) **METHOD FOR DETECTING A HORMONE DISRUPTING ACTIVITY OF ENVIRONMENTAL POLLUTANTS**
VERFAHREN ZUM NACHWEIS DER HORMONSTÖRENDEN WIRKUNG VON UMWELTVERSCHMUTZENDEN STOFFEN
PROCEDE DE MESURE DE L'ACTIVITE DESTRUCTRICE DE POLLUANTS DE L'ENVIRONNEMENT SUR UNE HORMONE

(30) Priority: 12.04.2000 NL 1014927
(43) Date of publication of application: 08.01.2003
(73) Proprietor: Meulenberg, Petronella Mariette Maria, NL-6546 MH Nijmegen (NL)
(72) Inventor: Meulenberg, Petronella Mariette Maria, NL-6546 MH Nijmegen (NL)
(74) Representative: De Hoop, Eric
(86) International application number: PCT/NL2001/000282
(87) International publication number: WO 2001/077688

(56) References cited:
- EP-A- 0 190 765
- WO-A-97/30353
- WO-A-97/31269
- WO-A-99/45388
- WO-A-99/45390
- B. J. DANZO: "Environmental xenobiotics may disrupt normal endocrine function by interfering with the binding of physiological ligands to steroid receptors and binding proteins." ENVIRONMENTAL HEALTH PERSPECTIVES, vol. 105, no. 3, 1997, pages 294-301, XP002085656 cited in the application
- K. W. GAIDO ET AL.: "Evaluation of chemicals with endocrine modulating activity in a yeast-based steroid hormone receptor gene transcription assay" TOXICOLOGY AND APPLIED PHARMACOLOGY, vol. 143, no. 1, 1997, pages 205-212, XP002085657

## Description

This invention relates to a method for detecting a hormone disrupting activity of environmental pollutants, and in particular to a detection using hormone transport proteins from blood. More specifically the invention relates to a competitive assay using one or more of the steroid or thyroid hormone transport proteins, wherein said proteins are being used as specifically binding proteins of high affinity to endogenous steroid or thyroid hormones in order to establish an affinity of xenobiotic substances and wherein a labelled steroid or thyroid hormone is being used as detection means.

### Background of the invention

The fact that the environment is more and more polluted by anthropogenic substances has for long been a reason for concern and in the past years this led to strict standards for maximum allowed substances in ground, water and air. On the basis thereof there are also extensive measuring and analysis systems for the detection and quantification of environmental pollutants. In the nineteen fifties it had already been found that certain substances, particularly DDT, have a hormone-like activity on organisms and since that time a large number of publications [1-6] have appeared on a hormone disrupting activity of various environment-polluting compounds, for instance pesticides, as well as natural substances, i.e. phytoestrogens. The large number of xenobiotic compounds occurring in the ground, water and food as well, combined with alarming information regarding gender disruptions of animals, a lower production of semen, reduced human fertility and an increase of gender related cancers has led to a widespread interest and extensive research into a possible hormone disrupting activity of xenobiotic substances. Especially the effect on human hormone systems is subject of many researches [7, 8] at the moment.

Human beings have various hormone systems, of which those involving steroid and thyroid hormones are the most important. Steroid and thyroid hormones for a large part determine the development, reproduction, metabolism and well-being of mankind [9]. Three main systems can be distinguished, namely the androgen/oestrogen system, the corticosteroid system and the thyroid gland hormone system. Said systems are regulated in the so-called HPTeA (Hypothalamus-Pituitary-Testis-Axis), the HPAA (Hypothalamus-Pituitary-Adrenal-Axis) and the HPTyA (Hypothalamus-Pituitary-Thyroid-Axis), that all show a daily rhythm and in which the synthesis of the final hormones is subject to a negative feedback.

### HPAA

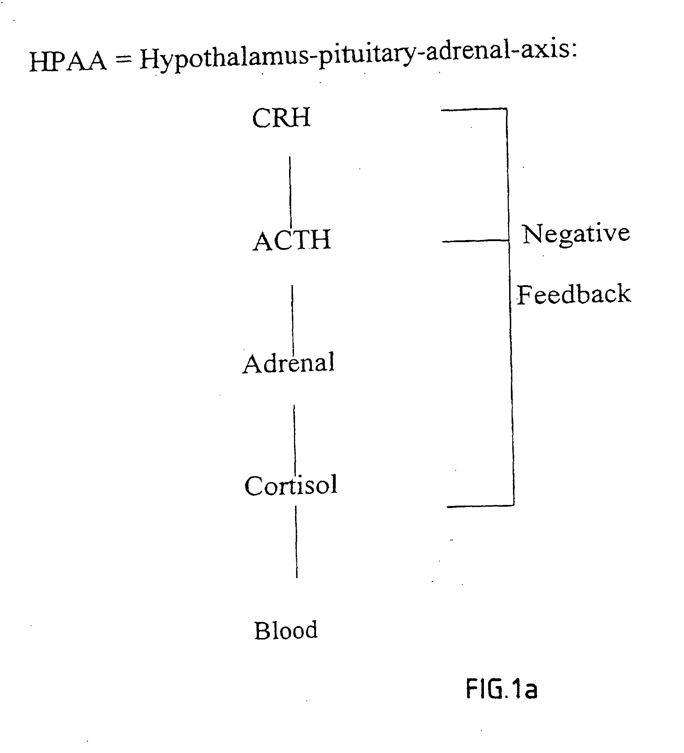

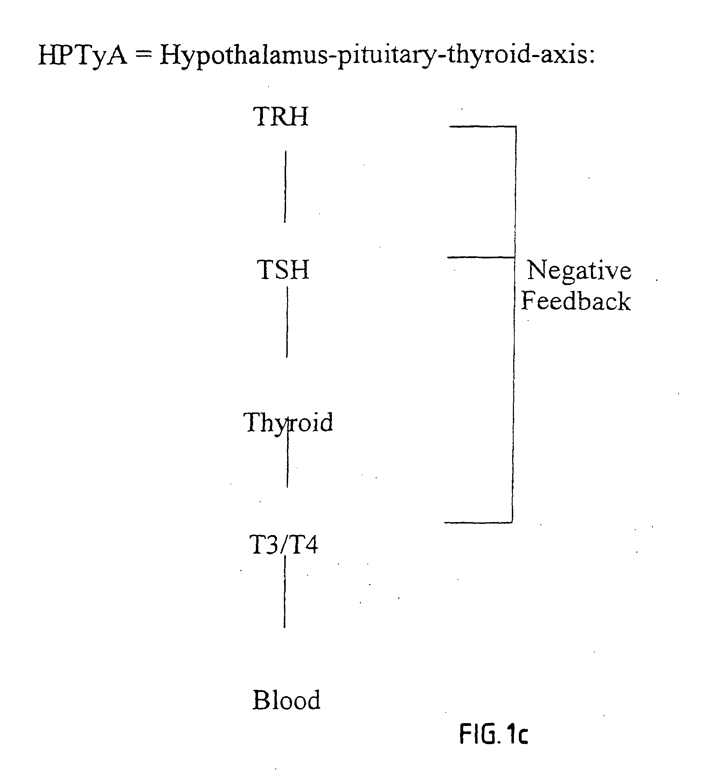

The HPAA regulates the synthesis of the corticosteroids of which cortisol is the most important. Cortisol is synthesized in the adrenal glands under the influence of ACTH (adrenocorticotroph hormone), which is secreted into blood by the hypophysis in reaction to CRH (Corticotrophin Releasing Hormone) from the hypothalamus. The HPAA also has a negative feedback effect. The concentration of cortisol normally is between certain limits and an increased concentration leads to a reduced secretion of ACTH, whereas a lowered concentration leads to an increased secretion of ACTH. This mechanism is schematically shown in Figure 1a. The mechanism is subject to a daily rhythm of high concentrations in the early morning and decreasing in the course of the day. The total concentration of cortisol, like testosterone, is divided over three fractions: bound to albumin, a protein of high capacity and relatively low affinity; bound to CBG (Corticosteroid Binding Globulin), the specific transport protein of low capacity and high affinity; and the unbound fraction. The division of the total quantity of cortisol over the three fractions is 6-10%, 90-95% and 2-4%, respectively [10].

### HPTeA

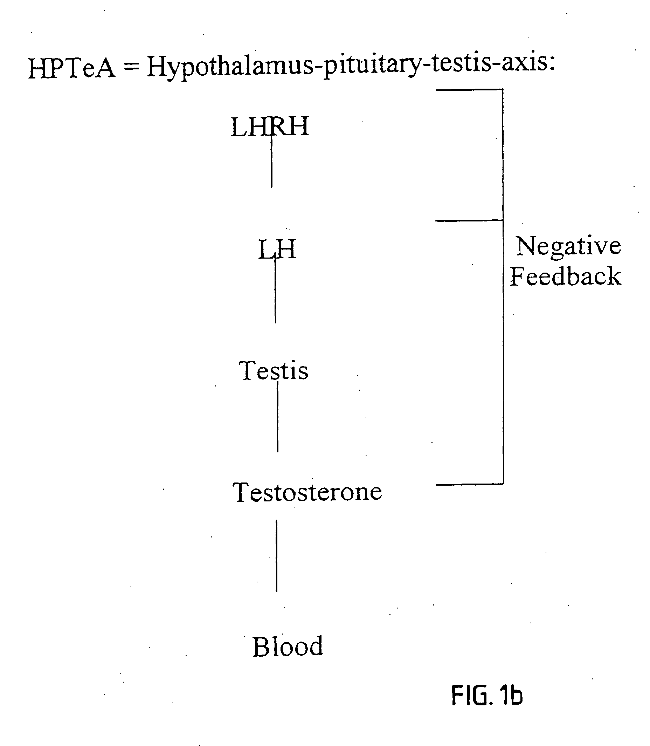

The HPTeA regulates the synthesis of androgens and oestrogens in the testes, ovaries and adrenal glands, wherein testosterone and oestradiol are the most important steroid sex hormones that greatly influence the sexual differentiation, reproduction and behaviour of mammals. The synthesis of testosterone in the testes is stimulated by LH (Luteinizing Hormone), that is secreted by the hypophysis in reaction to LHRH (Luteinizing Hormone Releasing Hormone) from the hypothalamus. The HPTeA functions in a daily rhythm of a high concentration in the morning and decreasing in the course of the day, the regulation also being subject to a negative feedback. This mechanism is schematically shown in figure 1b. After synthesis testosterone is secreted to the blood and in this way it reaches its target cells and target organs. Testosterone occurs in three forms in the blood: bound to albumin, a protein of high capacity and low affinity; bound to SHBG (Sex Hormone Binding Globulin), a specific protein of low capacity and high affinity; and unbound, i.e. the free fraction. The division of the total of testosterone in blood over the three fractions is 30-50%, 44-66% and 1-2%, respectively [11].

### HPTyA

The third important hormonal system comprises the thyroid hormones, of which T3 (tri-iodothyronine) and T4 (tetra-iodothyronine or thyroxine) are the most important compounds. The production of said hormones takes place in the thyroid gland. In the regulation via the HPTyA (see figure 1c) TRH (Thyrotrophin Releasing Hormone) is secreted by the hypothalamus and this stimulates the secretion of TSH (Thyroid Stimulating Hormone) from the hypophysis. TSH in its turn is the stimulus for the thyroid gland for synthesis of T3/T4 and the secretion of these hormones into the blood. Peripheral T4 is moreover converted into T3. The blood contains a very small fraction of the total concentration in the free from, namely 0.018-0.024% for T4, whereas the rest is divided over three proteins, namely 12-18% is bound to albumin having low affinity and high capacity; 73-82% is bound to TBG (Thyroxine Binding Globulin) having high affinity and low capacity; additionally there is an additional protein, TBPA (Thyroxin Binding Pre-Albumin), to which 6-9% of the T4 is bound [12]. TBPA is mainly involved in transport of T4 through the blood-brain-barrier. Normally the regulation of the HPTyA takes place via negative feedback and also in a daily rhythm comparable to the above-mentioned systems.

### Free Hormone Hypothesis

According to the Free Hormone Hypothesis the total concentration of steroid/thyroid hormones in blood is not decisive for the biological activity, the negative feedback and the daily rhythm, but mainly the non-protein-bound free fraction is [13-15]. Said free fraction is considered biologically active, because only unbound steroids are able to penetrate the membrane of target cells and subsequently bind to the corresponding receptors, followed by their effects on DNA level. Although there are indications that SHBG- and CBG-bound steroids are also capable of penetrating the target cells via SHBG- or CBG receptors, respectively, this mechanism works via the stimulation of "second messengers" in the cell [16, 17].

### Transport proteins

From their synthesis location, steroid and thyroid hormones divided over the three above-mentioned groups, are transported to their target organs/cells via the blood. The specific proteins binding with high affinity -SHBG, CBG and TBG- have the function to protect hormones against degradation by enzymes present, to transport them to their target locations and to form a kind of buffer, when quick supply of a certain compound is necessary. The synthesis of all three transport proteins takes place in the liver and is stimulated by oestrogens [18, 19].

### CBG

The fact that the hormonal status of human beings is determined by the concentration of the specific binding proteins in question, has been described. When, for instance the quantity of CBG drops, bound cortisol will be released and the concentration of the free fraction will rise strongly. This is the result of the ratio between CBG-bound cortisol and the free concentration, the latter being approximately 25 times lower. The opposite way is also the case; when the concentration of CBG rises, the concentration of the free cortisol will drop and moreover to a much higher degree. In the normal situation a drop or rise in the free cortisol is set off by the negative feedback system. That means that a rise of the free cortisol will lead to a drop in the secretion of ACTH and as a result a lowered synthesis of cortisol in the adrenal gland. The opposite case also occurs. For instance during pregnancy or the use of contraceptives an increased concentration of the endogenous or exogenic oestrogens, respectively, will lead to stimulation of the synthesis of CBG in the liver [11]; as a result relatively more cortisol is bound in the blood and the free fraction will drop; as a result the secretion of ACTH is stimulated and this in turn causes an increased cortisol synthesis until the free fraction will fall within the physiological range again. As a result increased concentrations of total cortisol will be found in the blood during pregnancy and the use of contraceptives, whereas the free concentrations are within the regular range. A pathogenic situation may arise when the total concentration of cortisol rises without compensation by an increase of CBG. This for instance occurs in Cushing's syndrome, when a constant production of cortisol takes place under the influence of a tumour [20]. A comparable situation, but with less outspoken symptoms can be found in depressed patients. The characterizing symptoms of hypercortisolemia are amongst others, obesity, striae, depression, a lowered functioning of the immune system, and a change in the metabolism, homoeostasis and behaviour [9]. Also insulin resistance, eating behaviour, HDL cholesterol and the risk of cardiovascular diseases are related to increased cortisol [21, 22].

### SHBG

SHBG has the same function as CBG, that is to say that it ensures transport of androgens/oestrogens, it protects these steroids against degradation and it forms a kind of buffer for quick supply. SHBG determines the androgenic status in human beings and a change in its concentration has strong effects on the free fraction of the androgens/oestrogens. Diseases associated to an increased or lowered concentration of for instance testosterone are hypogonadism in men and virilism in women, respectively [23]. Breast cancer and testicular cancer are also related to testosterone and oestradiol [24]. One of the former therapies against cancers caused by sex hormones was administering synthetic oestrogens. As a result the concentration of SHBG was increased and consequently the free fraction of testosterone was lowered.

SHBG is a protein consisting of two identical monomeric sub units. It has one binding site for steroids, situated in between the two monomers [25]. The most important and biologically most active steroids that bind with the highest affinity to SHBG are DHT, testosterone and oestradiol. Said compounds will in some cases compete for said one binding site. However, because the affinity of testosterone is approximately three times higher than that of oestradiol, oestradiol will be displaced from SHBG in case of a disruption of the balance by an increase of testosterone. In case of an increase of SHBG, more testosterone than oestradiol will be bound. In both situations this leads to a more oestrogenic environment in the blood.

As regards transport and protection, TBG also has the same function as the binding proteins discussed above. TBG also has one binding site, to which T3 and T4 both bind, the affinity of T4 being approximately 50 times higher than that of T3 [9]. Considering the extreme low free fractions of the thyroid hormones, each change in the concentration of TBG is immediately strongly manifested in a change of the free fraction. Under normal circumstances, the thyroid hormones regulate the basal metabolism, growth, development, neuronal balance and behaviour in human beings. Disorders and diseases associated with deviating concentrations are for instance Hashimoto's Disease, an auto-immune disease comprising antibodies against TBG and increased T4 concentrations; in stress increased T3-values are found and obesity and mental retardation have been associated with lowered T4 concentrations [9, 26].

### Effect of xenobiotic, hormone disrupting substances

Xenobiotic substances may enter the body through ingestion, inhalation or through the skin. When a substance has entered in the body and particularly the blood, it may show hormone-like effects. Various publications have already come out on substances having an oestrogenic effect, that show affinity to the oestrogen receptor [27, 28]. Although such an affinity can either be agonistic or antagonistic, substances have already been found that have a clear oestrogenic effect on cells in vitro and even on DNA level. Receptors, however, are situated in the cell and before a xenobiotic substance is able to reach a certain receptor, the substance will quite likely be transported through the blood. In this compartment, as explained above, the transport proteins occur. In case of pesticides there are structural and/or physical-chemical similarities between endogenous thyroids/steroids and some pesticides, such as for instance size or molecular weight, polarity, structure etc. Additionally there are pesticides that are used both in agriculture and -with some modification- in therapeutics, such as for instance conazolen [29]. A xenobiotic compound may show affinity or binding to one or more of the transport proteins. In that case endogenous hormones may be displaced therefrom, as a result of which the free concentration thereof increases with the above-mentioned consequences. In this way a xenobiotic compound may show an oestrogenic, androgenic, corticosteroid or thyroid effect after it has been taken up in the blood.

The concept of binding or affinity to specific transport proteins has already been described and is based on the pharmacology. When namely new pharmaceutics are tested for intended effects and possible side-effects, its behaviour in the blood is also established. An important factor here is the metabolic clearance and this for a large part depends on the binding of substances to various proteins. An example of this are the components of oral contraceptives.

As regards binding to blood proteins assays using SHBG and CBG have already been developed before [30-31]. These assays were either based on total serum or on the protein in question isolated from serum/plasma on Concanavaline A coupled to a solid carrier such as Sepharose. For detecting a binding, use was made of a radiolabelled ligand such as tritiated oestradiol, tritiated testosterone, tritiated DHT etc. The affinity of a compound that may or may not be endogenous was then determined on the basis of the quantity of displaced or bound labelled compound.

Such an assay has been taken over by Danzo (1997, 1998) [32, 33] to determine the binding/affinity to SHBG of xenobiotic substances. The SHBG source was post partum serum. ³H-5-alpha-DHT was used for the detection and the competition by xenobiotic substances was detected using the active carbon method followed by either radioactivity count or saccharose gradient centrifugation. It was shown that for instance hexachloro-cyclohexane, o,p'-DDT, pentachlorophenol and nonylphenol showed binding to SHBG. On the other hand, such behaviour was not found for methoxychlor, atrazine, p,p'-DDT, p,p'-DDE and dieldrin.

Déchaud et al. (1998) [34] used the method in which SHBG from serum of normal men and women was bound to Concanavaline A-Sepharose. Binding and competition studies were carried out here using tritiated testosterone and tritiated oestradiol. Additionally the binding was also considered in total serum with xenoestrogens, in which ammonium sulphate precipitation and radioimmunoassay were used to detect non-SHBG-bound testosterone. In these studies it was found that particularly phytoestrogens have affinity to SHBG, although much lower than the one of the endogenous steroids testosterone and oestradiol, followed by alkylphenols, bisphenol-A and phthalates. The concentrations of xenoestrogens used by these researchers were very high, however, and not relevant for environmental analyses. A drawback of both methods mentioned above is that because of the use of total serum a disruption may occur by other proteins present that have binding capacity to steroid or thyroid hormones. It is known that albumin is a protein of a high capacity that is capable of binding very many different substances. Moreover, SHBG also has affinity to the corticosteroids and conversely, CBG also has affinity to androgens/oestrogens. Additionally, the occurrence of a specific oestradiol binding protein in plasma of pregnant persons has been described by Englebienne (1986) [36]. The presence of non-relevant proteins having binding properties renders the methods described partially non-specific and less sensitive.

Apart from the effect of an increase of the free concentration of endogenous hormones, the binding of a xenobiotic substance to a blood protein may also reduce the availability of this substance to cellular receptors, as a result of which a possible hormone disrupting effect is reduced again. Not only xenobiotic substances, however, may have a hormone disrupting effect. In this connection plant components, particularly phytoestrogens are mentioned as well. Adlercreutz et al. (1997) [35] examined the effect of food components such as lignans and phytoestrogens on oestrogens and SHBG. From their results it appears that there is a positive significant correlation between these substances and the SHBG concentration, whereas there is a negative significant correlation between the substances and the percentage of free oestradiol in plasma. Phytoestrogens may possibly also show an oestrogenic effect on various other levels: receptor, SHBG and cell level.

Xenobiotic substances may accordingly have a agonistic or antagonistic hormonal effect. It is for instance described that PCBs show a structural similarity to T3/T4 and that they bind to the thyroid receptor and TBG, resulting in a displacement of endogenous thyroid hormones leading to an increased metabolic clearance of thyroid hormones. Regarding a thyroid-like effect, PCBs, dioxin and polyhalogenated aromatic hydrocarbons (PHAH) have been called neurotoxic. PHAHs have also been mentioned in connection with thyroid related problems in fish eating communities in the Great Lakes region in the USA. An anti-thyroid effect has also been described for flavonoids. As regards binding of phytoestrogens to TBG, however, nothing is known.

### Summary of the invention

In order to show a hormone disrupting effect of xenobiotic substances at the level of specific transport proteins, according to the invention a competitive binding assay has been developed, comprising a transport protein as most important specific binding component for the substances to be measured, and an antibody specifically generated against said transport protein to selectively isolate said transport protein, whereby the drawbacks from the prior art are overcome.

The invention accordingly provides a method for detecting a hormone disrupting activity of environmental pollutants, comprising binding a steroid or thyroid hormone transport protein from blood of a mammal to a solid carrier via a specific antibody to the transport protein, and subsequently incubating the solid carrier with a corresponding labelled hormone and the substance to be tested for a sufficiently long period of time in order to let competition of the labelled hormone and the substance to be tested for binding sites on the transport protein take place, wherein a measure for the hormone disrupting activity is obtained on the basis of the degree of displacement of the labelled hormone from the transport protein.

The method according to the invention is an improvement over the existing methods as to specificity and sensitivity. SHBG, CBG, TBG can be used as transport protein. The binding and/or affinity of xenobiotic substances can be detected by means of the displacement of for instance a labelled endogenous compound such as oestradiol, testosterone, DHT, T3, T4 etc. Radioisotopes (for instance ³H, ¹²⁵I, ³⁵P, ³⁵S etc.), enzymes (HRP, alkaline phosphatase, glucose-oxidase, etc.), fluorescent, bioluminescent, chemiluminescent labels, biotin and the like can be used as label.

The binding proteins can be isolated from mammal blood, for instance from human or animal blood; they can be commercially obtained; they can be produced *in vitro* using suitable cell lines; or they can be produced with bacteriophages using the phage display technique.

The competitive assay can be suitably carried out in microtiter plates. However, other solid carriers are also possible, such as Sepharose, agarose, nitrocellulose, nylon membrane, tubes, glass balls or synthetic balls etc. In case of for instance microtiter plates, the protein can be adsorbed to the wall of the wells and the labelled compound can be made to compete for binding sites with the substance to be measured. After incubation either the bound or the unbound fraction is measured. The measurement takes place according to the labelled compound used. Radio active substances are for instance measured in a gamma or beta counter. Fluorescent or luminescent compounds can be measured using a fluorescence or chemiluminescence-microtiter plate reader. Enzyme coupled compounds can for instance be spectrophotometrically measured after adding a suitable substrate.

The degree of binding and/or affinity is determined on the basis of a calibration curve comparable to those used in immunoassay. By adding increased concentrations of a compound of known affinity/binding as standard the decrease in binding of the labelled substance is determined. The signal measured then is inversely proportional to the concentration of the standard. Each substance to be analyzed can subsequently be related to said standard. Testosterone, oestradiol or DHT can for instance be used for SHBG. Cortisol, corticosterone or progesterone can be used for CBG. T3 or T4 can be used for TBG.

According to the invention the binding protein is not directly adsorbed to the wells of the microtiter plate, but via a specific antibody to the transport protein. For instance antibodies against SHBG, CBG or TBG may be used. Such antibodies are commercially available. The adsorption of antibodies on microtiter plates is well-known in the art of immunoassay. Such an embodiment is particularly suitable when blood, serum or plasma is used as source of the transport proteins. As a result of the specificity of the antibodies in question, other proteins such as albumin and the like will not bind. After the formation of the antibody/transport protein complex the competitive assay can be carried out on it as described above.

Another possible embodiment is the one in which a biotin labelled antibody is bound to streptavidin plates. Yet another embodiment is the one in which a conjugate of for instance cortisol, corticosterone, progesterone, testosterone, DHT, oestradiol, T3, T4 is adsorbed with a protein such as for instance BSA, OVA, thyro globulin, KLH and the like, on microtiter plate and the competitive reaction between the substance to be measured and the corresponding binding protein are carried out on it. The detection can then be made using a second, labelled antibody against the binding protein.

A further embodiment is a competitive assay, in which the antibody and the transport protein are coupled to magnetic particles and the complex formed after incubation with the substance to be tested and the labelled hormone is isolated using a strong magnet.

As exemplary embodiment a corticosteroid activity of any substance to be measured can be shown in this way using CBG and labelled cortisol/corticosterone/progesterone; an oestrogenic or androgenic activity using SHBG and oestradiol/testosterone/DHT; and a thyroid activity using TBG and T3/T4. Accordingly in the field of transport proteins, a possible hormone disrupting activity of xenobiotic or natural substances can be detected. An example of an SHBG assay for establishing a possible oestrogenic activity of substances is given below.

### Description of the Figures

Figure 1 schematically shows the most important hormonal systems in human beings.
   Sub figure 1a: HPAA = Hypothalamus-Pituitary-Adrenal-Axis; CRH = Corticotrophin Releasing Hormone; ACTH = Adrenocorticotrophic Hormone; Adrenal = adrenal gland.
   Sub figure 1b: HPTeA = Hypothalamus-Pituitary-Testis-Axis; LHRH = Luteinizing Hormone Releasing Hormone; LH = Luteinizing Hormone.
   Sub figure 1c: HPTyA = Hypothalamus-Pituitary-Thyroid-Axis; TRH = Thyrotrophin Releasing Hormone; TSH = Thyroid Stimulating Hormone; T3 = tri-iodothyronine; T4 = tetra-iodothyronine or thyroxine.
Figure 2 shows the standard curve of testosterone. The values are the averages of 11 measurements. Microtiter plates were coated with polyclonal anti-SHBG antibody (DAKO); after incubation the plates were incubated with 50 µl ³H-E2 and 50 µl testosterone; after incubation 75 µl was taken from the supernatant fluid and its radioactivity (cpm) was counted; the bound fraction was calculated on the basis of the total number of added cpm; in the graph the percentage of B/Bo (bound divided by bound without testosterone) is plotted out against the concentration of testosterone.
Figure 3 shows the comparison in terms of displacement of tritiated E2 of various pesticides. Plotted out is the bound fraction as a percentage of the total quantity of added ³H-E2 against the concentration of the test compound. Testosterone is included as reference.
Figure 4 is comparable to Figure 3, but for some pharmaceutical products.

### Example: SHBG assay for detecting an oestrogenic activity of xenobiotic substances

The SHBG assay was developed for detecting a possible oestrogenic activity of xenobiotic substances. As main component use was made of SHBG from serum of pregnant volunteers in the third trimester of pregnancy. Additionally use was made of commercially available polyclonal anti-SHBG antibody (DAKO) to isolate SHBG from serum on a microtiter plate. For the detection of binding of unknown substances use was made of tritiated oestradiol ([6.7-³H]-17-beta-oestradiol; by Amersham Life Science, Roosendaal, the Netherlands). As standard testosterone (Sigma-Aldrich B.V., Zwijndrecht, the Netherlands) was used in a concentration series. The reason for this combination was that the affinity of testosterone to SHBG is approximately 3 times higher than that of oestradiol. Bound tritiated oestradiol will therefore be easily displaced by testosterone (affinity of testosterone for SHBG is 1.6x10⁷ M⁻¹, of oestradiol 0.7x10⁻⁹ M⁻¹; see Dunn et al. 1981). In this way a sufficiently high sensitivity can be obtained to be able to measure testosterone in low concentrations.

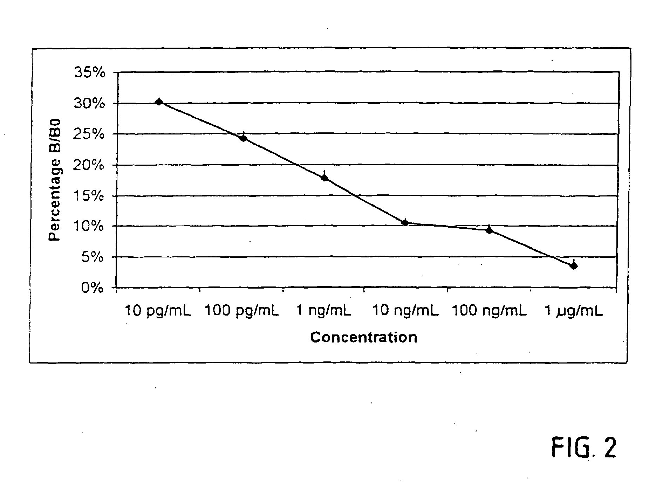

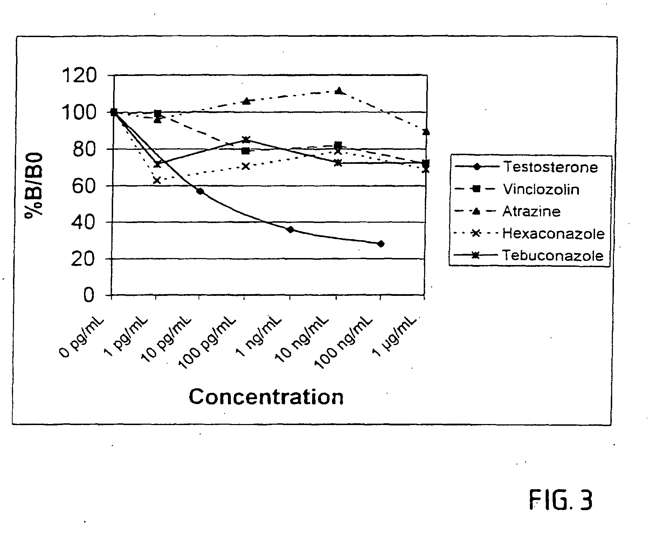

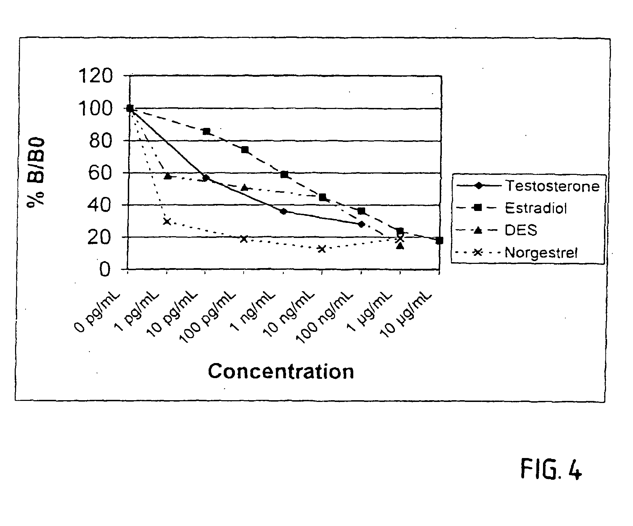

In Figure 2 the standard curve for testosterone is shown as an average of 11 measurements. On the basis of this curve a detection limit was calculated of 10 pg/ml on 90% binding. All compounds to be analyzed are related to the testosterone standard curve. For the analysis of an oestrogenic effect various compounds were tested in the SHBG assay, including endogenous steroids, pharmaceutical substances, pesticides and industrial waste products (alkyl phenols). The tested substances are shown in Table 1, in which also a score for the displacement of tritiated oestradiol is mentioned. By way of illustration an example of displacement curves of some pharmaceutical products is shown in Figure 3. In Figure 4 an example is shown of displacement curves of some pesticides. Additionally some combinations of pesticides and/or pharmaceutical substances were tested in the assay and from this it appeared that no absolute additive effect is found. The final result is comparable to the one of the strongest displacing substance, in which the additional substance has little to no effect.

**Table 1:**

| Scores for the oestrogenic activity of various tested substances. | | | | | |
|---|---|---|---|---|---|
| Classification | Tested substance | Activity | Classification | Tested Substance | Activity |
| Pesticides | Permethrin | + + | Pharmaceutics | Testosterone | + + |
| | Aldicarb | +/- | | Oestradiol | + + |
| | Dichlofluanide | + | | Norgestrel | + + |
| | Atrazine | - | | DES | + + |
| | Simazine | + /- | | Dehydro-iso- | + |
| | 2.4-D | + | | andosterone | |
| | Vinclozolin | + | | Andosterone | + |
| | Hexaconazole | +/- | | 6-alpha- | + |
| | Tebuconazole | +/- | | methylprednisolone | |
| | Glyphosate | +/- | | | |
| | Malathion | + | | | |
| Classification | Tested substance | Activity | | | |
| Industrial waste | Nonylphenol | + + | | | |
| products | Bisphenol-A | + + | | | |

| | | | | | |
|---|---|---|---|---|---|
| Elucidation: + + = strong displacement; + = moderate displacement; | | | | | |
| - = no displacement; +/- = no unambiguous result | | | | | |

### Discussion of the results

From Figure 3 and 4 and Table 1 it appears that there clearly are differences in the behaviour of the substances tested. For instance from the group of pesticides a clear displacement of oestradiol is found for permethrin, followed by malathion, vinclozolin, 2.4-D and dichlofluanide. On the other hand, atrazine shows no activity at all, whereas for the other pesticides the results are not unambiguous. Permethrin belongs to the group of pyrethroids and an oestrogenic effect for some of these compounds, although not for permethrin, was reported by Eil and Nisula (1990) [37]. This can be explained by the fact that these researchers used testosterone as reference, as a result of which permethrin may possibly have to be used in higher concentrations so as to be able to detect a displacement. Of vinclozolin it has already been described that it has an oestrogenic effect (Kelce et al., 1994) [38]; also 2.4-D and malathion are described as pesticides of potential oestrogenic activity (Denneman et al., 1998 [5]; Van Dooren en Kaiser, 1997) [39]. Little information about dichlofluanide is known in literature, but Van Dooren [39] attributed no hormone disrupting effect to this compound. The lack of oestrogenic activity of atrazine corresponds with the results of Danzo (1997) [32]; however, it appears that atrazine does show an activity at oestrogen receptor level. Of the other pesticides with an ambiguous result (aldicarb, simazine, hexaconazole, tebuconazole and glyphosate) simazine and glyphosate are described as having influence on the reproductive system (Van Dooren and Kaiser, 1997) [39].

As regards the pharmaceutical compounds it appears that DES and norgestrel have high affinity to SHBG. For norgestrel this was expected on the basis of the information described by Pugeat et al. (1981) [40]. The effect of DES on the other hand is quite unexpected, as this substance would have its oestrogenic effect through the oestrogen receptor. Regarding the other substances no data about a possible binding to SHBG are available.

The last group, the one of the alkylphenols nonylphenol and bisphenol-A, shows a remarkably strong effect in the SHBG assay and this result entirely corresponds with the results of Déchaud et al. (1998) [34] and confirms the oestrogenic activity of alkylphenol compounds as reported by Toppari et al. (1996) [2], Daston et al. (1997) [3] and Danzo (1998).

It can be concluded that using the SHBG assay described herein, a hormone disrupting or oestrogenic disrupting activity of environmental pollutants can be detected. As such the SHBG assay can therefore be used in the analysis of the toxicity of environmental pollutants. Moreover it is good addition to the much used oestrogen receptor assay, because in this way additional information can be obtained at a different level of oestrogenic activity.

### Further uses

It will be clear that apart from the tested compounds that are pesticides, alkylphenols and pharmaceutical substances, numerous other environmental pollutants can be measured. Moreover natural substances as well, such as for instance phytoestrogens and other food components can be analyzed. Because mammals and particularly human beings are in practice exposed to many different natural and xenobiotic substances, it is also relevant to determine the effects of various combinations of two or more substances, including pesticides, phytoestrogens, pharmaceuticals, veterinary drugs, and industrial waste products, in the various measuring systems. The substances to be measured may occur in waste water, drinking-water, surface water, groundwater, rainwater, snow, sediments, soil samples, agricultural products, horticultural products, foodstuffs, clinical samples etc. As regards aqueous samples, in most cases they can be directly used in the assay. Other samples may have to undergo a pretreatment such as fluid-fluid, solid-fluid, SPE or HPLC. Possible matrix effects in the assay should also be taken into account in these cases.

Furthermore the assays can also be coupled to common analytical methods. When for instance the hormone disrupting activity has to be detected of an unknown sample, said sample can be tested in one or more of the assays discussed. In case of a positif signal the various components of the sample can be further analyzed using for instance HPLC. After said analysis the various peaks separated by HPLC can be measured again in the said assays on hormone disrupting activity. Unknown peaks can also be further analyzed using GC-MS or LC-MS.

An on-line system can be developed by for instance producing an affinity column in which one or more of the transport proteins is coupled to a solid carrier. A liquid sample, particularly an aqueous sample, is then led over this column and all the substances binding to said transport proteins in a next step are eluated from the column and led to an analytic detection system, such as for instance an HPLC or LC-MS. In this way unknown compounds having affinity to one of the transport proteins can be identified.

### References

[1] T. Colborn, D. Dumanoski and J.P. Myers, Our Stolen Future, 2nd ed., Little, Brown & Comp., London, 1997
[2] J. Toppari, J.Chr. Larsen, P. Christiansen, A. Giwercman, P. Grandjean, L.J. Gluillett Jr., B. Jégou, T.K. Jensen, P. Jouannet, N. Keiding, H. Leffers, J.A. McLachlan, O.Meyer, J. Müller, E. Pajpert-De Meyts, T. Scheike, R. Sharpe, J. Sumpter and N.E. Skakkebaek, Environm. Health Persp. 104, (1996) 741.
[3] G.P. Daston, J.W. Gooch, W.J. Breslin, D.L. Shuey, A.I. Nikiforov and J.W. Gorsuch, Reprod. Toxicol. 11 (1997) 465.
[4] B. Jimenez, Trends Anal. Chem. 16 (1997), 596.
[5] W.D. Denneman, N. Heeg, A.J. Palsma and H.M.J. Janssen Xenooestrogenen en drinkwater(bronnen), RIWA Rapport, July 1998.
[6] Kurzer & Xu (1997)
[7] B.J. Danzo, Cell. Mol. Life Sci. 54 (1998) 1249.
[8] H. Adlercreutz, Environm. Toxicol. Pharmacol. 7 (1999) 201.
[9] R. Hall, J. Anderson, G.A. Smart and M. Besser, Fundamentals of Clinical Endocrinology, 3rd ed., Pitman Medical Ltd. Kent, 1980, pp 94-207.
[10] P. Meulenberg, Thesis, Wageningen Agricultural University, 1995.
[11] J.P. Dunn, B.C. Nisula and D. Rodbard, J. Clin. Endocrinol. Metab. 53 (1981) 58.
[12] W.M. Partridge, Am. J. Physiol. 252 P1 (1987) E157.
[13] C.M. Mendel, Endocr. Rev. 10 (1989) 232.
[14] P.K. Siiteri, J.T. Murrai, G.L. Hammond, J.A. Nisker, W.J. Raymoure and R.W. Kuhn, Rec. Prog. Hormone Res. 38 (1982) 457.
[15] R. Ekins, Endocr. Rev. 11 (1990) 5.
[16] N. Fortunati, J. Endocrin. Invest. 22 (1999) 223.
[17] W. Rosner, M.S. Kahn, N.A. Romas and D.J. Hryb, in: Binding Proteins of Steroid Hormones, M.G Forest & M. Pugeat, ed., John Libbey, London, 1986, page 567.
[18] D.C. Anderson, Clin. Endocrinol. 3 (1974) 69.
[19] A. Vermeulen, in: Binding Proteins of Steroid Hormones, M.G Forest & M. Pugeat, ed., John Libbey, London, 1986, page 383.
[20] P.J. Trainer and A. Grossman, Clin. Endocrin. 34 (1991) 317.
[21] J.W. Funder, Corticosteroid Receptors in the Brain, in: Brain Endocrinology, M. Motta ed., Raven Press, Ltd., New York, 1991, page 133.
[22] R. Fraser, M.C. Ingram, N.H. Anderson, C. Morrison, E. Davies and J.M.C. Connell, Hypertension 33 (1999) 1364.
[23] P. Johnson, Clin. Sci. 66 (1984) 369.
[24] P. Englebienne, Anticancer Res. 32 (1989) 574.
[25] P.H. Petra, J. Steroid Biochem. Molec. Biol. 40 (1991) 735.
[26] E.S. Sher, X.M. Xu, P.M. Adams, C.M. Craft and S.A. Stein, Toxicol. Indust. Health 14 (1998) 121.
[27] K. Rehman, K.W. Schramm and A.A. Kettrup, Chemosphere 38 (1999) 3303.
[28] T. Zacharewski, Environm. Sci. Technol. 31 (1997) 613.
[29] A.J. Vidal Puig et al., Eur. J. Endocrinol. 130 (1994) 333.
[30] P.F. Bruning, J.M.G. Bonfrèr and W.J. Nooyen, Clin. Chim. Acta 148 (1985) 69.
[31] J.M.G. Bonfrèr, P.F. Bruning and W.J. Nooyen, J. Steroid Biochem. 33 (1989) 69.
[32] B.J. Danzo, Environ. Health Perspect. 105 (1997) 294.
[33] B.J. Danzo, Cell. Mol, Life Sci. 54 (1998) 1249.
[34] H. Déchaud, C. Ravard. F. Claustrat, A. Brac de la Perrière and M. Pugeat, Steroids 64 (1999) 328.
[35] H. Adlercreutz, K. Höckerstedt, C. Bannwart, S. Bloigou, E. Hämäläinen, T. Fotsis and A. Ollus, J. Steroid Biochem. 27 (1987) 4.
[36] P. Englebienne, Clin, Chem. 32 (1986) 574.
[37] C.Eil and B. Nisula, J. Steroid Biochem. (1990) 409.
[38] W.R. Kelce et al., Toxicol. Appl. Pharmacol. 126 (1994) 276.
[39] Van Dooren and Kaiser, Rapport van de Alternatieve Consumenten-bond, Uitg. Primavera, Amsterdam, 1997.
[40] M. Pugeat et al., J. Clin. Endocr. Metab. 53 (1981) 69.

## Claims

1. A method for detecting a hormone disrupting activity of environmental pollutants, comprising binding a steroid or thyroid hormone transport protein from blood of a mammal to a solid carrier via a specific antibody to the transport protein, and subsequently incubating the solid carrier with a corresponding labelled hormone and the substance to be tested for a sufficiently long period of time in order to let competition of the labelled hormone and the substance to be tested for binding sites on the transport protein take place, wherein a measure for the hormone disrupting activity is obtained on the basis of the degree of displacement of the labelled hormone from the transport protein.

2. A method according to claim 1, wherein the transport protein is selected from corticosteroid binding globulin (CBG), sex hormone binding globulin (SHBG) or thyroxine binding globulin (TBG).

3. A method according to claim 2, wherein the transport protein originates from human or animal blood.

4. A method according to any one of the preceding claims, wherein the labelled hormone has been labelled by a signal producing label, such as a radioisotope, enzyme, fluorescent label, bio- or chemiluminescent label; or wherein a signal enhancing system is being used such as the biotin-(strept)avidin system together with one of the above-mentioned labels.

5. A method according to any one of the preceding claims, wherein the competitive reaction is carried out in an indirect manner, wherein a conjugate of a specifically binding hormone for the transport protein and a non-specific protein is bound to a solid carrier and the competitive reaction between the transport protein and the substance to be measured is carried out on the conjugate bound onto the solid phase, after which the complex is detected by means of a labelled antibody against the said transport protein.

6. A method according to claim 1, comprising:
- coating the wells of microtiter plates with anti-SHBG antibody;
- incubating the plates with human serum and subsequent extensive washing of the plates;
- adding ³H-oestradiol together with a compound to be tested to the washed plates and incubating for a sufficiently long period of time to let competition of both components for binding sites take place;
- removing a part of the supernatant fluid from the wells of the plate and measuring the radioactivity of the samples;
- calculating the amount of radioactivity bound in the wells of the plate on the basis of the amount of radioactivity in the samples and the total added radioactivity;
- determining the degree of displacement of ³H-oestradiol from the binding sites in the wells by the tested compound.

7. A kit for detecting a hormone disrupting activity of environmental pollutants, comprising:
- a solid carrier coated with antibody against a steroid or thyroid hormone transport protein;
- a quantity of transport protein;
- a corresponding labelled hormone;
- a reference hormone for producing a calibration curve;
- possible buffers for making dilutions of the labelled hormone and the reference hormone.

## Patentansprüche

1. Verfahren zum Nachweis einer hormonstörenden Wirkung von umweltverschmutzenden Stoffen, umfassend das Binden eines Transportproteins für Steroid- oder Thyroidhormone aus Blut von Saugtieren via einen spezifischen Immunkörper für das Transportprotein auf einem festen Träger, darauf Inkubieren des festen Trägers mit einem entsprechend markierten Hormon und dem zu prüfenden Stoff während einer genügend langen Zeit um Konkurrenz des markierten Hormons und des zu prüfenden Stoffes für Bindungsstellen auf dem Transportprotein stattfinden zu lassen, wobei ein Maß für die hormonstörende Wirkung an Hand des Maßes von Verdringung des markierten Hormons vom Transportprotein erhalten wird.

2. Verfahren nach Anspruch 1, wobei das Transportprotein aus Corticosteroid Bindungs Globulin (CBG), Sex Hormon Bindungs Globulin (SHBG) oder Thyroxin Bindungs Globulin (TBG) ausgewählt wird.

3. Verfahren nach Anspruch 2, wobei das Transportprotein aus menschlichem oder tierischem Blut stammt.

4. Verfahren nach einem der vorhergehenden Ansprüche, wobei das markierte Hormon mit einem signalproduzierenden Markierungsstoff markiert ist, wie einem Radioisotop, Enzyme, einem fluoreszierenden Merkstoff, einem bio- oder chemilumineszierenden Merkstoff; oder wobei ein signalverstärkendes System wie das biotin- (strept)avidin-System zusammen mit einem der obenerwähnten Markierungsstoffe benutzt wird.

5. Verfahren nach einem der vorhergehenden Ansprüche, wobei die konkurrierende Reaktion auf indirekte Weise ausgeführt wird, wobei ein Konjugat eines spezifisch bindenden Hormons für das Transportprotein und eines aspezifischen Proteins auf einem festen Träger gebunden wird und die konkurrierende Reaktion zwischen dem Transportprotein und dem zu messenden Stoff auf dem auf der festen Phase gebundenen Konjugat ausgeführt wird, worauf der Komplex mit Hilfe eines markierten Immunkörpers gegen das betreffende Transportprotein nachgewiesen wird.

6. Verfahren nach Anspruch 1, umfassend:
- Beschichten der Brunnen von Mikrotiterplatten mit anti-SHBG Immunkörper;
- Inkubieren der Platten mit menschlichem Serum und darauf ausführlich Waschen der Platten;
- Zufügen von ³H-Estadriol zusammen mit einer zu prüfenden Verbindung an den gewaschenen Platten und Inkubieren während einer genügend langen Zeit um Konkurrenz von beiden Komponenten für Bindungsstellen stattfinden zu lassen;
- Entfernen eines Teils der obenstehenden Flüssigkeit aus den Brunnen der Platte und Messen der Radioaktivität der Proben;
- Berechnen der Menge der in den Brunnen der Platte gebundenen Radioaktivität an Hand der Menge Radioaktivität in den Proben und der gesamt zugefügten Radioaktivität;
- Bestimmen des Maßes von Verdringung von ³H-Estradiol von den Bindungsstellen in den Brunnen durch die geprüfte Verbindung.

7. Kitt zum Nachweis einer hormonstörenden Wirkung von umweltverschmutzenden Stoffen, umfassend:
- einen festen Träger mit Immunkörper gegen ein Transportprotein für Steroid- oder Thyroidhormone bekleidet;
- eine Menge von Transportprotein;
- ein entsprechend markiertes Hormon;
- ein Referenzhormon zum Produzieren einer Eichkurve;
- eventuelle Puffer zum Machen von Verdünnungen des markierten Hormons und des Referenzhormons.

## Revendications

1. Procédé de détection de l'activité destructrice de polluants de l'environnement sur une hormone, comprenant la liaison d'une protéine de transport d'une hormone stéroïdienne ou thyroïdienne du sang d'un mammifère à un porteur solide par l'intermédiaire d'un anticorps spécifique dirigé contre la protéine de transport, et par la suite l'incubation du porteur solide avec une hormone marquée correspondante et la substance à doser pendant une période de temps suffisamment longue pour permettre la compétition de l'hormone marquée et de la substance à doser pour les sites de liaison sur la protéine de transport, procédé dans lequel une mesure de l'activité destructrice sur l'hormone est obtenue sur la base du degré de déplacement de l'hormone marquée à partir de la protéine de transport.

2. Procédé selon la revendication 1, dans lequel la protéine de transport est choisie parmi la globuline fixant les corticostéroïdes ( Corticosteroid Binding Globulin,CBG), la globuline fixant les hormones sexuelles (Sex Hormone Binding Globulin, SHBG) ou la globuline fixant la thyroxine (Thyroxine Binding Globulin, TBG).

3. Procédé selon la revendication 2, dans lequel la protéine de transport provient de sang humain ou animal.

4. Procédé selon l'une quelconque des revendications précédentes, dans lequel l'hormone marquée a été marquée par un marqueur producteur de signal, tel qu'un isotope radioactif, une enzyme, un marqueur fluorescent, un marqueur bioluminescent ou chimioluminescent ; ou dans lequel un système d'amplification du signal est utilisé tel qu'un système biotine (strept)avidine avec un des marqueurs susmentionnés.

5. Procédé selon l'une quelconque des revendications précédentes, dans lequel la réaction compétitive est réalisée d'une manière indirecte, dans lequel un conjugué d'une hormone liant spécifiquement la protéine de transport et une protéine non spécifique est lié à un porteur solide et la réaction compétitive entre la protéine de transport et la substance à mesurer est réalisée sur le conjugué lié sur la phase solide, après quoi le complexe est détecté au moyen d'un anticorps marqué contre ladite protéine de transport.

6. Procédé selon la revendication 1, comprenant à :
- l'enduction des puits de plaques de microtitrage avec un anticorps anti-SHBG ;
- L'incubation des plaques avec du sérum humain, puis le lavage extensif des plaques ;
- l'ajout de ³H-oestradiol avec le composé à doser sur les plaques lavées et l'incubation pendant une période de temps suffisamment longue pour permettre la compétition des deux composants pour les sites de liaison ;
- l'extraction d'une partie du surnageant des puits de la plaque et la mesure de la radioactivité des échantillons ;
- le calcul de la quantité de radioactivité liée dans les puits de la plaque sur la base de la quantité de radioactivité dans les échantillons et de la radioactivité ajoutée totale ;
- la détermination du degré de déplacement du ³H-oestradiol à partir des sites de liaison dans les puits par le composé dosé.

7. Kit de détection de l'activité destructrice de polluants de l'environnement sur une hormone, comprenant :
- un porteur solide enduit d'un anticorps dirigé contre une protéine de transport d'une hormone stéroïdienne ou thyroïdienne ;
- une quantité de protéine de transport ;
- une hormone marquée correspondante ;
- une hormone de référence pour produire une courbe d'étalonnage ;
- des tampons appropriés pour faire des dilutions de l'hormone marquée et l'hormone de référence.
